# EUROPÄISCHE PATENTSCHRIFT

(11) **EP 2 588 158 B1**
(45) Veröffentlichungstag und Bekanntmachung des Hinweises auf die Patenterteilung: **03.10.2018**
(21) Anmeldenummer: 11730212.5
(22) Anmeldetag: 27.06.2011
(51) Int. Cl.: A61M 1/16, A61M 1/36

(54) **MEDIZINISCHE FUNKTIONSEINRICHTUNG, PROZESSFLUID UND MEDIZINISCHE BEHANDLUNGSVORRICHTUNG**
MEDICAL FUNCTIONAL DEVICE, PROCESS FLUID, AND MEDICAL TREATMENT APPLIANCE
DISPOSITIF FONCTIONNEL MÉDICAL, FLUIDE DE TRAITEMENT ET APPAREIL DE TRAITEMENT MÉDICAL

(30) Priorität: 29.06.2010 DE 102010025516
(43) Veröffentlichungstag der Anmeldung: 08.05.2013
(73) Patentinhaber: Fresenius Medical Care Deutschland GmbH, 61352 Bad Homburg (DE)
(72) Erfinder: BRANDL, Matthias, 97631 Bad Königshofen (DE); HERRENBAUER, Michael, 61267 Neu-Anspach (DE); WEHMEYER, Wolfgang, 72076 Tübingen (DE)
(74) Vertreter: Bobbert & Partner Patentanwälte PartmbB
(86) Internationale Anmeldenummer: PCT/EP2011/003152
(87) Internationale Veröffentlichungsnummer: WO 2012/000637

(56) Entgegenhaltungen:
- WO-A1-02/062454
- WO-A1-03/043680
- DE-C1- 10 011 208
- US-A- 4 211 597
- US-A- 4 661 246
- US-A- 5 423 738
- US-A- 5 893 382
- US-A1- 2005 131 332

## Beschreibung

Die vorliegende Erfindung betrifft eine medizinische Funktionseinrichtung gemäß Anspruch 1. Sie betrifft ferner ein Verfahren gemäß Anspruch 13 sowie eine medizinische Behandlungsvorrichtung gemäß Anspruch 14. Aus der Praxis ist bekannt, Blut führende Einrichtungen zur extrakorporalen Blutbehandlung, wie Leitungen, Filter, Kassetteneinheiten von Dialysemaschinen und dergleichen, vor ihrem Einsatz in einem Blutbehandlungsprozess mit einem Prozessfluid, bei welchem es sich nicht um Blut handelt, zu primen, zu spülen oder zu befüllen. Beispielsweise offenbaren die DE 100 11 208 C1, US 5,893,382, WO 03/043680 A1 und WO 02/062454 A1 verschiedene Hämofiltrationsgeräte, welche vor ihrem Einsatz mit einem Prozessfluid gespült oder befüllt werden.
Eine Aufgabe der vorliegenden Erfindung ist, eine weitere medizinische Funktionseinrichtung vorzuschlagen, mittels welcher ein Primen oder Spülen und/oder Befüllen eines extrakorporalen Blutkreislaufs unter Einsatz eines Prozessfluids möglich ist.
Diese Aufgabe wird durch eine medizinische Funktionseinrichtung mit den Merkmalen des Anspruchs 1 gelöst, durch ein Verfahren mit den Merkmalen des Anspruchs 13 sowie eine medizinische Behandlungsvorrichtung mit den Merkmalen des Anspruchs 14.

Alle oder manche mit der erfindungsgemäßen medizinischen Funktionseinrichtung erzielbaren Vorteile lassen sich ungeschmälert auch mit der erfindungsgemäßen medizinischen Behandlungsvorrichtung erzielen.
Erfindungsgemäß wird eine interne oder externe medizinische oder medizintechnische Funktionseinrichtung vorgeschlagen, welche wenigstens einen Prozessfluidkreislauf oder Abschnitte desselben aufweist, welche jeweils vorgesehen sind zum Aufnehmen eines Prozessfluids. Die medizinische Funktionseinrichtung weist ferner wenigstens eine erste Filtereinrichtung und wenigstens eine zweite Filtereinrichtung auf, welche jeweils innerhalb des Prozessfluidkreislaufs oder eines Abschnitts hiervon angeordnet sind.
Die medizinische Funktionseinrichtung ist vorgesehen und ausgestaltet zur einmaligen Verwendung in einem Verfahren zum extrakorporalen Behandeln des Bluts eines Patienten. Sie ist als Disposable, als Einwegartikel, als Wegwerfartikel oder dergleichen ausgestaltet, vorgesehen und/oder vermarktet sein.
Das erfindungsgemäße Verfahren betrifft das Betreiben einer erfindungsgemäßen medizinischen Funktionseinrichtung und umfasst ein vollständiges oder teilweises Öffnen einer zweiten Ventileinrichtung und/oder einer okkludierenden Pumpeinrichtung zum Reinigen der ersten Filtereinrichtung.

Die erfindungsgemäße medizinische Behandlungsvorrichtung ist vorgesehen und konfiguriert zum Aufnehmen und/oder Ansteuern wenigstens einer erfindungsgemäßen medizinischen Funktionseinrichtung und/oder zum Durchführen des erfindungsgemäßen Verfahrens.

Vorteilhafte Weiterbildungen der vorliegenden Erfindung sind jeweils Gegenstand der Unteransprüche.

Erfindungsgemäße Ausführungsformen können einige oder alle der folgenden Merkmale in beliebiger Kombination aufweisen.

Die medizinische Behandlungsvorrichtung ist zu ihrer Verwendung in extrakorporalen Blutbehandlungsverfahren vorgesehen.

Die medizinische Behandlungsvorrichtung ist in manchen erfindungsgemäßen Ausführungsformen eine Blutreinigungsvorrichtung wie eine Hämodialysiervorrichtung, eine Hämofiltrationsvorrichtung, eine Hämodiafiltrationsvorrichtung. Sie kann eine Vorrichtung zum Durchführen von Leberersatzverfahren, eine Vorrichtung zum Durchführen von Immunadsorption oder dergleichen sein.

Der Begriff "Verfahren zum extrakorporalen Behandeln des Bluts eines Patienten", im Folgenden auch als "extrakorporales Blutbehandlungsverfahren" bezeichnet, bezeichnet ein Verfahren, welches dem extrakorporalen Behandeln von Blut dient.

Vor Durchführen des extrakorporalen Blutbehandlungsverfahrens, mittels welchem die extrakorporale Behandlung des Bluts erfolgen soll, können eine Mehrzahl oder Vielzahl einzelner Prozesse oder Prozessschritte erforderlich sein, wie das Spülen oder Primen und/oder Befüllen des extrakorporalen Blutkreislaufs, unter Einsatz eines Prozessfluids.

Ein in einem Prozess des extrakorporalen Blutbehandlungsverfahrens eingesetztes Prozessfluid kann vorgesehen sein, verschiedene Funktionen zu übernehmen, wie beispielsweise als Spül- und/oder als Befüllfluid, als Verdrängungsfluid oder als Sterilisationsfluid und dergleichen, zu dienen.

Bei dem Prozessfluid kann es sich um ein Fluid allgemein, vorzugsweise um eine Flüssigkeit oder eine Kombination oder Mischung verschiedener Fluide allgemein oder Flüssigkeiten handeln.

Das Prozessfluid kann mittels oder unter Wirkung einer Fördereinrichtung in den Prozessfluidkreislauf eingebracht und/oder in diesem gefördert werden.

Eine solche - erste - Fördereinrichtung kann im Prozessfluidkreislauf als Teil der medizinischen Funktionseinrichtung oder als hierzu externe Fördereinrichtung vorgesehen sein. Die Fördereinrichtung muss nicht Teil der Funktionseinrichtung sein.

Die Fördereinrichtung ist in bestimmten Ausführungsformen ausgewählt aus Membranpumpen, peristaltischen Pumpen und/oder Impellerpumpen.

In bestimmten Ausführungsformen der vorliegenden Erfindung ist die Fördereinrichtung im Prozessfluidkreislauf angeordnet, um Prozessfluid von einer Prozessfluidquelle in den Prozessfluidkreislauf zu fördern.

In bestimmten Ausführungsformen weist die erfindungsgemäße medizinische Funktionseinrichtung wenigstens eine Fördereinrichtung auf, welche in oder auf der medizinischen Funktionseinrichtung, vorzugsweise als Teil hiervon, im Prozessfluidkreislauf angeordnet ist, um das Prozessfluid im Prozessfluidkreislauf oder in einem Abschnitt hiervon zu fördern.

Diese - zweite - Fördereinrichtung kann in oder auf der medizinischen Funktionseinrichtung, in bevorzugten Ausführungsformen als Teil derselben, im Prozessfluidkreislauf angeordnet sein. Die zweite Fördereinrichtung kann als Teil der medizinischen Funktionseinrichtung, insbesondere einer Disposable-Funktionseinrichtung, zum Einmalgebrauch ausgestaltet und vorgesehen sein.

Der extrakorporale Blutkreislauf oder Abschnitte desselben können in bestimmten erfindungsgemäßen Ausführungsformen der medizinischen Funktionseinrichtung mit der medizinischen Funktionseinrichtung gekoppelt oder verbunden sein oder werden oder einen Teil der medizinischen Funktionseinrichtung bilden.

In manchen Ausführungsformen der erfindungsgemäßen medizinischen Funktionseinrichtung können Abschnitte des extrakorporalen Blutkreislaufs, wie beispielsweise Kanäle, Rohrabschnitte, Leitungen oder Leitungsabschnitte, integral bzw. einstückig mit der medizinischen Funktionseinrichtung ausgestaltet sein oder anderweitig mit der medizinischen Funktionseinrichtung verbunden sein.

Der extrakorporale Blutkreislauf kann neben Leitungen, wie beispielsweise einer arteriellen und einer venösen Patientenleitung Schläuche, Schlauchsysteme, Kanäle, Ventile, Behandlungseinrichtungen wie beispielsweise Filter- und/oder Dialysiereinrichtungen, Fördereinrichtungen und dergleichen aufweisen.

Der Begriff "Prozessfluidkreislauf", wie er hierin verwendet wird, bezeichnet ein Fluidsystem oder eine Fluidanordnung, welche(s) dazu geeignet und vorgesehen ist, Prozessfluide aufzunehmen und von diesem durchströmt zu werden. Die Prozessfluide können zum Zwecke eines Prozesses wie Spülen, Primen, Substituieren, Senken von Schadstoffkonzentrationen und dergleichen zum Einsatz kommen.

Der Prozessfluidkreislauf kann vollständig oder abschnittsweise Teil der erfindungsgemäßen medizinischen Funktionseinrichtung sein.

In manchen Ausführungsformen der erfindungsgemäßen medizinischen Funktionseinrichtung können Abschnitte des Prozessfluidkreislaufs integral mit der medizinischen Funktionseinrichtung ausgestaltet sein bzw. in die medizinische Funktionseinrichtung integriert sein.

In bestimmten Ausführungsformen sind prozessfluidführende Abschnitte des Prozessfluidkreislaufs wie Leitungen, z.B. in Form von Schläuchen, Teil der medizinischen Funktionseinrichtung.

Der Prozessfluidkreislauf kann Leitungen, Schläuche, Schlauchsysteme, Kanäle, Ventile, Drosseln, Filtereinrichtungen, Sensoren, Kammern, Ausbuchtungen, Einrichtungen oder Räume oder Bereiche zum Speichern oder Vorhalten oder Zurückhalten von Prozessfluiden sowie Steuer- oder Regeleinrichtungen zum Steuern oder Regeln eines Strömungsdurchflusses der Prozessfluide, Fördereinrichtungen zum Fördern der Prozessfluide und dergleichen aufweisen oder aus beliebigen Kombinationen hiervon bestehen.

Der Prozessfluidkreislauf kann ein geschlossener Kreislauf sein. Der Prozessfluidkreislauf kann jedoch auch Teil eines (übergeordneten) Fluidkreislaufs sein. Der Prozessfluidkreislauf kann ein offener Kreislauf sein.

In bestimmten Ausführungsformen der vorliegenden Erfindung ist die medizinische Funktionseinrichtung zu ihrem Einsatz mit wenigstens einer Prozessfluidquelle verbindbar oder verbunden.

Die Prozessfluidquelle kann - ggf. zusammen mit einer Aufnahmeeinrichtung für gebrauchtes Prozessfluid - Teil einer Prozessfluidversorgungseinheit sein.

Bei einer Prozessfluidquelle kann es sich um eine Vorrichtung zur Online-Herstellung, d.h. für eine während des extrakorporalen Blutbehandlungsverfahrens erfolgende Herstellung, von Prozessfluid handeln. Die Prozessfluidquelle kann jedoch auch eine Quelle sein, welche Prozessfluide batchweise oder portioniert zur Verfügung stellt, beispielsweise ein mit Prozessfluid oder Vorstufen hiervon gefüllter Beutel und dergleichen.

Kombinationen verschiedener Prozessfluidquellen zur Bereitstellung eines oder mehrerer verschiedener Prozessfluide sind von der Erfindung gleichfalls umfasst.

In bestimmten erfindungsgemäßen Ausführungsformen ist die Prozessfluidquelle in Strömungsrichtung des Prozessfluids stromaufwärts der ersten Filtereinrichtung in einem ersten Leitungsabschnitt des Prozessfluidkreislaufs angeordnet. In solchen Ausführungsformen ist vorgesehen, das Prozessfluid von der Prozessfluidquelle der ersten Filtereinrichtung des Prozessfluidkreislaufs zuzuführen.

Um ein Strömen des Prozessfluids in den Prozessfluidkreislauf und/oder innerhalb des Prozessfluidkreislaufs zu unterbrechen bzw. zu unterbinden (oder auch nur zu reduzieren) und/oder freizugeben, können in manchen erfindungsgemäßen Ausführungsformen geeignete strömungsverringernde oder - unterbindende Einrichtungen wie Ventileinrichtungen, Durchflusssperreinrichtungen, wie Absperrhähne, Klemmen, und dergleichen vorgesehen sein. Ergänzend oder alternativ können ein Strömen zulassende bzw. eine Strömung freigebende Einrichtungen oder weitere Fördereinrichtungen neben den genannten Fördereinrichtungen vorgesehen sein.

In bestimmten erfindungsgemäßen Ausführungsformen der medizinischen Funktionseinrichtung ist wenigstens eine Ventileinrichtung im Prozessfluidkreislauf angeordnet. In bestimmten Ausführungsformen ist die Ventileinrichtung stromabwärts der zweiten Filtereinrichtung angeordnet. In solchen Ausführungsformen kann ein Strömen des Prozessfluids von der zweiten Filtereinrichtung im Prozessfluidkreislauf zurück zur Prozessfluidquelle oder in deren Richtung verhindert werden.

Die Ventileinrichtung ist in bestimmten Ausführungsformen der vorliegenden Erfindung dazu ausgelegt und/oder vorgesehen, einen Durchfluss des Prozessfluids von der zweiten Filtereinrichtung durch den weiteren Prozessfluidkreislauf zu verhindern, vorzugsweise zumindest vorübergehend und gezielt.

In solchen Ausführungsformen ist die Ventileinrichtung dazu vorgesehen und ausgelegt, einen Leitungsabschnitt des Prozessfluidkreislaufs stromabwärts der zweiten Filtereinrichtung teilweise oder vollständig zu blockieren.

Optional, zum Beispiel in Ausführungsformen mit Kaskadenfiltration, kann zusätzlich wenigstens eine weitere Ventileinrichtung neben der vorstehend angegebenen, d.h. - in einem Fall wenigstens zweier Ventileinrichtungen - ersten, Ventileinrichtung im Prozessfluidkreislauf vorgesehen sein.

In bestimmten erfindungsgemäßen Ausführungsformen der medizinischen Funktionseinrichtung ist die weitere, d.h. in diesem Fall zweite, Ventileinrichtung stromabwärts der ersten Filtereinrichtung angeordnet. In solchen Ausführungsformen kann ein Rückströmen des Prozessfluids aus einer Drain-Leitung in den Prozessfluidkreislauf zur ersten Filtereinrichtung und/oder in umgekehrter Richtung verhindert werden.

In bestimmten Ausführungsformen ist - alternativ oder ergänzend zur Ventileinrichtung stromabwärts der zweiten Filtereinrichtung und/oder zur Ventileinrichtung stromabwärts der ersten Filtereinrichtung - eine Fördereinrichtung, insbesondere eine Pumpe zur Proportionalregelung, stromabwärts der zweiten Filtereinrichtung im Prozessfluidkreislauf angeordnet.

In bestimmten Ausführungsformen ist stromabwärts der ersten Filtereinrichtung eine zweite Ventileinrichtung und/oder eine okkludierende Pumpeinrichtung vorgesehen. Diese kann z. B. in einer Drain-Leitung vorgesehen sein. Mittels dieser Anordnung kann durch deren Öffnen eine Reinigung der ersten Filtereinrichtung vorgehen und betrieben werden.

Die medizinische Funktionseinrichtung kann funktionell an eine medizinische Blutbehandlungsvorrichtung koppelbar sein oder gekoppelt an diese vorliegen.

Eine funktionelle (An-)Kopplung der medizinischen Funktionseinrichtung an die medizinische Behandlungsvorrichtung kann dazu dienen, eine Ansteuerung der medizinischen Funktionseinrichtung gleichwelcher Art, beispielsweise mechanisch, pneumatisch, elektrisch, elektronisch und/oder zur Datenübertragung, zu erreichen.

Die medizinische Behandlungsvorrichtung weist in manchen erfindungsgemäßen Ausführungsformen eine oder mehrere Steuer- oder Regeleinrichtungen, wie interne oder externe Steuer- oder Regeleinrichtungen, und/oder Aktoren auf, die vorgesehen und konfiguriert sind, um wenigstens eine erfindungsgemäße medizinische Funktionseinrichtung zu steuern oder zu regeln.

In bestimmten Ausführungsformen der vorliegenden Erfindung ist die medizinische Funktionseinrichtung dazu vorgesehen und/oder konfiguriert, um bei ihrer Ankopplung oder Verbindung mit einer medizinischen Behandlungsvorrichtung derart angesteuert zu werden, dass durch entsprechendes Schalten wenigstens der Ventileinrichtung stromabwärts der zweiten Filtereinrichtung im Prozessfluidkreislauf und/oder Betätigen wenigstens einer der Fördereinrichtungen im Prozessfluidkreislauf das Einleiten von Prozessfluid in den Prozessfluidkreislauf und/oder den extrakorporalen Blutkreislauf veranlassbar ist.

In bestimmten Ausführungsformen der vorliegenden Erfindung ist die medizinische Funktionseinrichtung dazu vorgesehen und/oder konfiguriert, um bei ihrer Ankopplung oder Verbindung mit einer medizinischen Behandlungsvorrichtung derart angesteuert zu werden, dass durch entsprechendes Schalten wenigstens der ersten Ventileinrichtung stromabwärts der zweiten Filtereinrichtung und der zweiten Ventileinrichtung stromaufwärts der ersten Filtereinrichtung im Prozessfluidkreislauf und/oder Betätigen wenigstens einer der Fördereinrichtungen im Prozessfluidkreislauf das Einleiten von Prozessfluid in den Prozessfluidkreislauf und/oder den extrakorporalen Blutkreislauf veranlassbar ist.

Jede der Ventileinrichtungen kann unabhängig von der Ausgestaltung anderer Ventileinrichtungen in den entsprechenden Ausführungsformen derart vorgesehen und angeordnet sein, um - durch entsprechende Schaltung/Ansteuerung durch die Steuer- oder Regeleinrichtung der medizinischen Blutbehandlungsvorrichtung oder manuell - einen Prozessfluidfluss entlang oder innerhalb des Prozessfluidkreislaufs zu unterbrechen oder freizugeben.

Jede der Ventileinrichtungen kann unabhängig von der Ausgestaltung anderer Ventileinrichtungen in den entsprechenden Ausführungsformen derart vorgesehen und angeordnet sein, um - durch entsprechende Schaltung/Ansteuerung durch die Steuer- oder Regeleinrichtung der medizinischen Blutbehandlungsvorrichtung oder manuell - einen Prozessfluidfluss durch die zweite Filtereinrichtung in den extrakorporalen Blutkreislauf zuzulassen.
In bestimmten Betriebszuständen der medizinischen Funktionseinrichtung ist ein Übertritt oder Übergang des Prozessfluids aus dem Prozessfluidkreislauf in den extrakorporalen Blutkreislauf vorgesehen oder beabsichtigt. Der Übergang des Prozessfluids aus dem Prozessfluidkreislauf in den extrakorporalen Blutkreislauf kann in bzw. innerhalb oder mittels einer Filtereinrichtung, insbesondere der zweiten Filtereinrichtung, erfolgen.
Der Begriff "Filtereinrichtung", wie er hierin verwendet wird, bezeichnet allgemein eine Einrichtung, welche - vollständig oder abschnittsweise - zum Filtern, d.h. Reinigen, ggf. Sterilisieren und dergleichen, des Prozessfluids ausgelegt ist. Die in der erfindungsgemäßen medizinischen Funktionseinrichtung vorgesehenen Filtereinrichtungen sind als Einwegteil ausgestaltet.

Die erste Filtereinrichtung ist erfindungsgemäß ein Sterilfilter. Die Verwendung eines Sterilfilters kann vorteilhaft dazu beitragen, die Sicherheit der Sterilität des Prozessfluids zu erhöhen.
Die erste Filtereinrichtung kann wie auch die zweite Filtereinrichtung (eine oder mehrere) Hohlfaserkapillarmembranen aufweisen und/oder ein Hohlfaserfiltermodul sein. Die erste Filtereinrichtung ist vollständig oder abschnittsweise in die erfindungsgemäße medizinische Funktionseinrichtung integriert bzw. integral mit dieser verbunden. Gleiches kann auch auf die zweite Filtereinrichtung zutreffen.
In bestimmten Ausführungsformen der erfindungsgemäßen medizinischen Funktionseinrichtung ist die zweite Filtereinrichtung eine Blutfiltereinrichtung des extrakorporalen Blutkreislaufs. Geeignete Blutfiltereinrichtungen schließen Filtereinrichtungen zum Durchführen einer Hämodialyse, einer Hämofiltration oder einer Hämodiafiltration ein.
Die erfindungsgemäße medizinische Funktionseinrichtung wird in bestimmten Ausführungsformen der vorliegenden Erfindung zum Spülen und/oder Befüllen des extrakorporalen Blutkreislaufs eingesetzt. In solchen Ausführungsformen kann das Prozessfluid bevorzugt eine Spüllösung, wie eine Dialysierflüssigkeit, eine Substitutionsflüssigkeit, eine Kochsalzlösung und dergleichen sein.
In bestimmten Ausführungsformen der vorliegenden Erfindung wird die erfindungsgemäße medizinische Funktionseinrichtung zur Blutrückgabe im Rahmen einer extrakorporalen Blutbehandlung und/oder zur Bolusgabe (z.B. von Medikamentenlösungen und dergleichen) während einer extrakorporalen Blutbehandlung eingesetzt und ist hierzu vorgesehen.
In bestimmten Ausführungsformen der vorliegenden Erfindung ist die medizinische Funktionseinrichtung vorgesehen und/oder konfiguriert, um derart angesteuert zu werden, dass sie - beispielsweise durch entsprechende maschinelle Ansteuerung - zwischen einem Spül- und/oder Befüll-Prozess und einem Blutbehandlungs-Prozess schaltbar ist.

Dies kann mit Hilfe einer Schaltung und/oder Ansteuerung der Ventile und/oder der Fördereinrichtung(en) erreicht werden.

In bestimmten Ausführungsformen der vorliegenden Erfindung dient der Spül- und/oder Befüll-Prozess dazu, die Blut führenden Leitungen des extrakorporalen Blutkreislaufs mit Spülflüssigkeit zu durchströmen, um so beispielsweise Luft aus den Leitungen auszupressen, Partikel auszuströmen und/oder die Benetzbarkeit des Leitungsinneren mit Blut zu verbessern. Der extrakorporale Blutkreislauf kann hierbei durch Spülen des Leitungsinneren und/oder des Inneren von Blut führenden Einrichtungen des extrakorporalen Blutkreislaufs vor seiner Befüllung mit Blut gereinigt werden.

In anderen erfindungsgemäßen Ausführungsformen kann das Prozessfluid eine Medikamentenlösung und/oder Infusionslösung wie beispielsweise eine Medikamenten- und/oder Infusionslösung zur Bolusgabe oder zur kontinuierlichen Gabe, und dergleichen sein. Beispiele hierfür schließen, ohne darauf beschränkt zu sein, eine Bolusgabe in den Blutkreislauf zur Volumenexpansion oder eine Bolusgabe zur Veränderung der Zusammensetzung des Blutplasmas, und dergleichen ein.

In anderen erfindungsgemäßen Ausführungsformen kann das Prozessfluid zur Blutrückgabe eingesetzt werden. Durch Übertritt des Prozessfluids über eine Membran der zweiten Filtereinrichtung in einen Blut führenden Abschnitt derselben kann nach Ende der Blutbehandlung das Blut aus dem extrakorporalen Blutkreislauf zum Patienten zurückgeführt werden. Das übertretende Prozessfluid verdrängt hierbei das Blut aus dem Blut führenden Abschnitt in einen venösen und/oder arteriellen Leitungsabschnitt des extrakorporalen Blutkreislaufs. In solchen Ausführungsformen können optische Detektoren an der arteriellen und/oder der venösen Patientenleitung vorgesehen sein, welche eine Phasengrenze zwischen Blut und Prozessfluid erkennen und ein Stoppen des Rückgabeprozesses veranlassen. Selbstverständlich können auch weitere Merkmale und/oder Formen der Blutrückgabe in Betracht gezogen werden, ohne die vorliegende Erfindung auf das vorstehend Ausgeführte zu beschränken.

Die erfindungsgemäße medizinische Funktionseinrichtung und das ihr zugrunde liegende Prinzip können vorteilhaft bei einer push-/pull-Hämodiafiltration sowie anderen push-pull-Verfahren eingesetzt werden. Hiermit kann es beispielsweise möglich sein, eine Lösung für den Volumenaustausch derartiger Verfahren bereitzustellen.

Der Übertritt oder Übergang des Prozessfluids aus dem Prozessfluidkreislauf in den extrakorporalen Kreislauf kann unter Erzeugen einer Druckdifferenz erreicht werden.

In bestimmten Ausführungsformen der vorliegenden Erfindung ist die Fördereinrichtung im Prozessfluidkreislauf (entspricht der vorstehend als zweite Fördereinrichtung bezeichneten Fördereinrichtung)als Teil der Funktionseinrichtung zwischen der ersten und der zweiten Filtereinrichtung angeordnet.

Die zweite Fördereinrichtung kann - neben der ersten Fördereinrichtung, welche vorwiegend zum Fördern des Prozessfluids aus der Prozessfluidquelle dient, dazu beitragen, den Übergang des Prozessfluids aus dem Prozessfluidkreislauf in den extrakorporalen Kreislauf zu fördern.

In bestimmten Ausführungsformen der vorliegenden Erfindung ist die zweite Fördereinrichtung vorgesehen und konfiguriert, um mittels eines Aktors der medizinischen Behandlungsvorrichtung betrieben zu werden.

Dabei ist der Aktor zum Antreiben bzw. Betreiben der zweiten Fördereinrichtung in manchen erfindungsgemäßen Ausführungsformen nicht als Einwegprodukt sondern wieder verwendbar, beispielsweise als Teil der medizinischen Funktionseinrichtung, ausgestaltet. Lediglich das Mittel, das in der medizinischen Funktionseinrichtung in stofflichem Kontakt mit dem Prozessfluid steht und dabei den Druckgradienten bewirkt, ist in manchen Ausführungsformen als Einwegteil ausgestaltet.

Die zweite Fördereinrichtung ist in bestimmten Ausführungsformen der erfindungsgemäßen medizinischen Funktionseinrichtung ausgewählt aus Membranpumpen, peristaltischen Pumpen und/oder Impellerpumpen.

Die medizinische Funktionseinrichtung ist erfindungsgemäß als Einwegartikel, d.h. Disposable, ausgestaltet. In bestimmten erfindungsgemäßen Ausführungsformen ist sie als Blutkassette, zum Beispiel als Einweg-Blutkassette, ausgestaltet.

In manchen Ausführungsformen kann die erfindungsgemäße medizinische Funktionseinrichtung, z.B. in Form einer Blutkassette, eine Vielzahl von Funktionen aufweisen, welche sämtlich oder teilweise zur Einmalverwendung vorgesehen sind. Solche Funktionen können z.B. Ventileinrichtungen, Pumpen, Luftabscheider, Bilanziersystem, Dialysatheizung usw. einschließen.

Ausführungsformen der vorliegenden Erfindung können einen oder mehrere der im Folgenden beschriebenen Vorteile aufweisen.

Die vorliegende Erfindung stellt vorteilhaft ein vereinfachtes System zum Einbringen von Prozessfluid in einen extrakorporalen Blutkreislauf im Rahmen eines extrakorporalen Blutbehandlungsverfahrens bereit.

Die in der medizinischen Funktionseinrichtung der vorliegenden Erfindung vorgesehene zweistufige Filtration des Prozessfluids kann das Risiko einer Kontamination des Prozessfluids oder von Blut führenden Abschnitten eines extrakorporalen Blutkreislaufs bei Ausfall eines Filters reduzieren und so vorteilhaft dazu beitragen, die Sicherheit des Systems zu erhöhen. Die Zweistufigkeit kann erfindungsgemäß nahezu ohne Zusatzaufwand über die Umkehrung des Filtratflusses in der zweiten Filtereinrichtung erreicht werden: Da der prozessfluidseitige Ausgang der zweiten Filtereinrichtung durch die zweite Ventileinrichtung ganz oder teilweise blockiert werden kann, kann das Prozessfluid durch einfaches Umschalten der zweiten Ventileinrichtung über die Membran in den Blut führenden Abschnitt der zweiten Filtereinrichtung geleitet werden. Auf diese Weise kann vorteilhaft einfach und wenig aufwendig eine Umkehrung der sonst während einer Filtration, z.B. Ultrafiltration, üblichen Filtrationsrichtung, in welcher dem Patientenblut normalerweise hier Flüssigkeit entzogen wird, erreicht werden.

Da die beiden Filtereinrichtungen als Bestandteile eines einzigen Einwegprodukts ausgestaltet sind, kann es in bestimmten Ausführungsformen vorteilhaft möglich sein, auf einen vorgeschalteten bzw. vorgelagerten Sterilfilter, welcher beispielsweise als stationärer Filter und damit zur Wiederverwendung vorgesehen ist, und einen damit verbundenen Integritätstest nach einer Behandlung zu verzichten.

Da es erfindungsgemäß vorteilhaft nicht mehr erforderlich ist, eine (bei wiederverwendbaren Filtereinrichtungen erforderliche) Integritätsmessung einer Filtereinrichtung durchzuführen zu müssen, kann vorteilhaft ein Verfahrensablauf der medizinischen Blutbehandlungsvorrichtung vereinfacht werden. Dies kann vorteilhaft dazu beitragen, die maschinellen und/oder manuellen Prozessschritte, die vor jeder Behandlung beim Aufrüsten des extrakorporalen Blutkreislaufs einer Dialysemaschine notwendig sein können, zu vereinfachen. Auf diese Weise kann es vorteilhaft möglich sein, ein im Wesentlichen vereinfachtes System bereitzustellen. Dies kann vorteilhaft dazu beitragen, die Sicherheit des Systems zu erhöhen.

Da es mit der erfindungsgemäßen medizinischen Funktionseinrichtung vorteilhaft möglich ist, zwei herkömmliche, zur Wiederverwendung bzw. mehrfachen Verwendung vorgesehene Filtereinrichtungen durch die erfindungsgemäße medizinische Funktionseinrichtung zu ersetzen, kann es vorteilhaft möglich sein, an Material- und/oder Kostenaufwand zu sparen.

Der oder die Filtereinrichtungen der erfindungsgemäßen medizinischen Einweg-Funktionseinrichtung kann/können in ihrer Größe an die zu erwartende Retentionskapazität für eine Behandlung angepasst werden, so dass es aufgrund dieser Optimierung vorteilhaft weiter möglich ist, Kosten einzusparen. Dies trifft vor allem auf die zweite Filtereinrichtung der medizinischen Funktionseinrichtung zu.

Daneben kann die erfindungsgemäße medizinische Funktionseinrichtung aufgrund der hierin als Einwegfilter vorgesehenen Filtereinrichtungen vorteilhaft dazu beitragen, die Gefahr einer Kontamination zu verringern. Dies kann in bestimmten erfindungsgemäßen Ausführungsformen ferner dazu beitragen, ausreichend sterile Bedingungen bzw. Qualitätskriterien einer Sterilisation für das Einbringen eines (ggf. zu Beginn nicht sterilen) Prozessfluids in den extrakorporalen Blutkreislauf einhalten zu können. So kann es zum Beispiel vorteilhaft möglich sein, eine Kontamination, insbesondere der Blut führenden Leitungen, zu vermeiden.

Im Folgenden wird die vorliegende Erfindung beispielhaft unter Bezugnahme auf die beigefügte Zeichnung beschrieben. In deren Figuren bezeichnen identische Bezugszeichen gleiche oder identische Elemente. Es gilt:
- Fig. 1: zeigt schematisch vereinfacht einen Abschnitt einer erfindungsgemäßen medizinischen Funktionseinrichtung gemäß einer ersten Ausführungsform; und
- Fig. 2: zeigt schematisch vereinfacht einen Abschnitt einer erfindungsgemäßen medizinischen Funktionseinrichtung gemäß einer zweiten Ausführungsform.

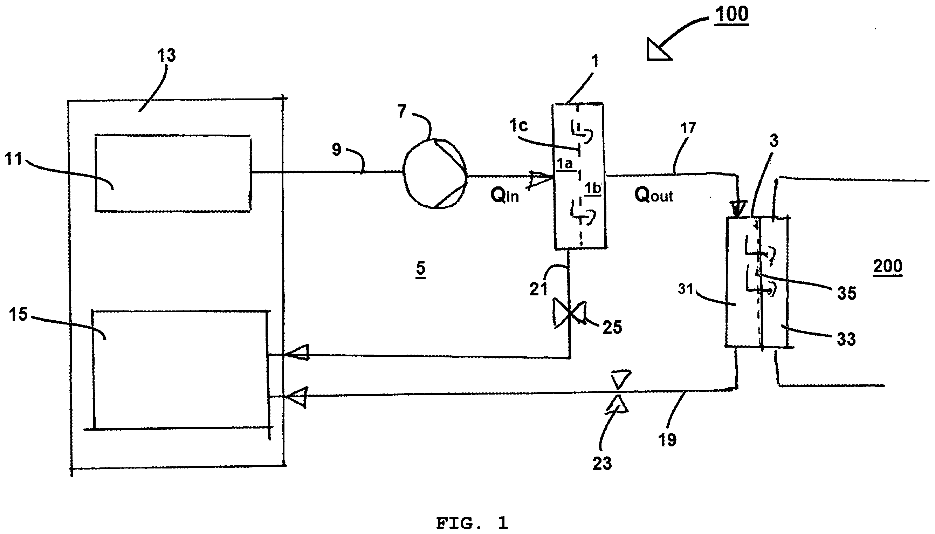

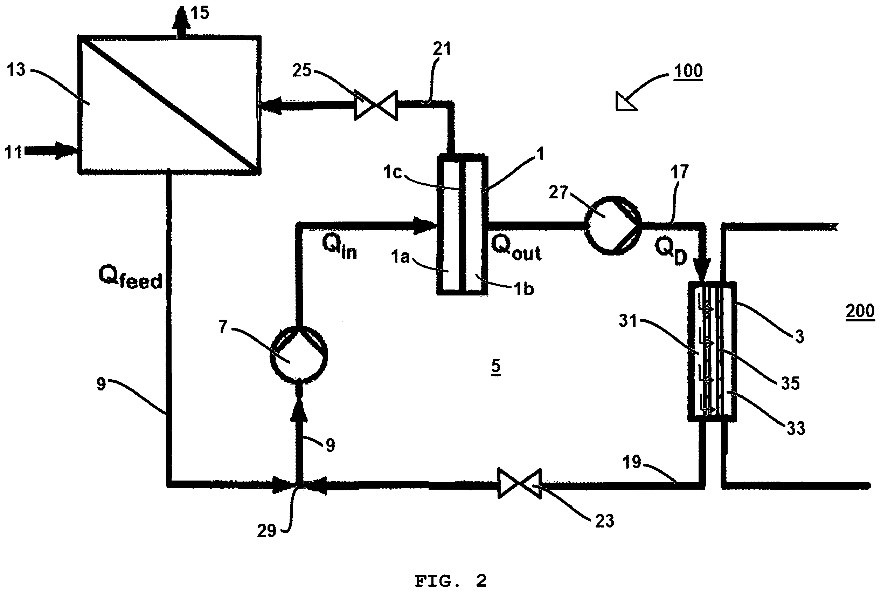

Die Begriffe "stromaufwärts" und "stromabwärts" im Zusammenhang mit bestimmten erfindungsgemäßen Ausführungsformen, wie sie auch im Zusammenhang mit den Fig. 1 und 2 verwendet werden, sind jeweils auf die mit Pfeilen angegebene Strömungsrichtung bezogen. Die Pfeilrichtungen entsprechen jeweils der Strömungsrichtung; die Pfeile zeigen jeweils stromabwärts.

**Fig. 1** zeigt einen Abschnitt einer erfindungsgemäßen medizinischen oder medizintechnischen Funktionseinrichtung 100 (kurz: Funktionseinrichtung) gemäß einer ersten Ausführungsform.

Die Funktionseinrichtung 100 kann ein Disposable mit zwei integrierten Filterstufen und integrierten Prozessfluid- und Blutprozesseinheiten sein.

Wie Fig. 1 zeigt, weist die medizinische Funktionseinrichtung 100 eine erste Filtereinrichtung 1 und eine zweite Filtereinrichtung 3 auf.

Die erste Filtereinrichtung 1 ist in manche erfindungsgemäßen Ausführungsformen ein Sterilfilter.

Die zweite Filtereinrichtung 3 ist in bestimmten erfindungsgemäßen Ausführungsformen - wie jener der Fig. 1 - ein Blutfilter.

Die zweite Filtereinrichtung 3 ist in einem extrakorporalen Blutkreislauf 200 angeordnet.

Die erste Filtereinrichtung 1 weist einen ersten Abschnitt 1a als Eingangsseite für Prozessfluid und einen zweiten Abschnitt 1b als Ausgangsseite für Prozessfluid auf.

Die zweite Filtereinrichtung 3 weist einen Prozessfluid führenden Abschnitt 31 und einen Blut führenden Abschnitt 33 auf.

Die Abschnitte 1a, 1b, der ersten Filtereinrichtung 1 und/oder die Abschnitte 31, 33 der zweiten Filtereinrichtung 3 können jeweils zwei oder mehr Kammern aufweisen. Die Kammern können durch Hohlfaserkapillarmembranen 1c bzw. 35 voneinander getrennt sein.

Die erste Filtereinrichtung 1 und die zweite Filtereinrichtung 3 sind Teil eines Prozessfluidkreislaufs 5.

Die erste Filtereinrichtung 1 und die zweite Filtereinrichtung 3 sind Einwegprodukte, d.h. sie werden nach ihrem einmaligen Gebrauch gemeinsam mit der medizinischen Funktionseinrichtung 100 verworfen.

Beide Filtereinrichtungen 1, 3 können vor ihrem einmaligen Einsatz auf Integrität getestet sein.

Vorteilhafterweise weist jede Filtereinrichtung 1, 3 eine für den angestrebten Zweck ausreichende Retentionskapazität auf.

Die Funktionseinrichtung 100 weist eine erste Fördereinrichtung 7 zum Fördern von Prozessfluid auf.

Die erste Fördereinrichtung 7 ist in der in Fig. 1 dargestellten Ausführungsform in einem ersten Leitungsabschnitt 9 des Prozessfluidkreislaufs 5 zwischen einer Prozessfluidquelle 11 und der ersten Filtereinrichtung 1 angeordnet.

Die Prozessfluidquelle 11 ist, wie hier gezeigt, Teil einer Prozessfluidverarbeitungseinheit 13, welche in der besonderen Ausführungsform der Fig. 1 neben der Prozessfluidquelle 11 eine Aufnahmeeinrichtung 15 für gebrauchtes Prozessfluid aufweist.

Der Prozessfluidkreislauf 5 umfasst neben dem ersten Leitungsabschnitt 9 (zwischen Prozessfluidquelle 11 und erster Filtereinrichtung 1) einen zweiten Leitungsabschnitt 17 (zwischen der ersten Filtereinrichtung 1 und der zweiten Filtereinrichtung 3), einen dritten Leitungsabschnitt 19, welcher zwischen der zweiten Filtereinrichtung 3 und der Aufnahmeeinrichtung 15 für gebrauchtes Prozessfluid angeordnet ist, sowie einen vierten Leitungsabschnitt 21, welcher zwischen der ersten Filtereinrichtung 1 und der Aufnahmeeinrichtung 15 für gebrauchtes Prozessfluid angeordnet ist.

In manchen Ausführungsformen der vorliegenden Erfindung stellt der Prozessfluidkreislauf 5 oder ein Teil hiervon einen geschlossenen Kreislauf dar.

Nicht alle Elemente, Abschnitte oder Bestandteile bzw. Teile des Prozessfluidkreislaufs 5 sind solche in jeder Ausführungsform auch der Funktionseinrichtung 100. Dies kann insbesondere für den dritten Leitungsabschnitt 19, vorzugsweise inklusive der Fördereinrichtung 7, und den vierten Leitungsabschnitt 21 zutreffen.

Im dritten Leitungsabschnitt 19 ist stromabwärts der zweiten Filtereinrichtung 3 eine erste Ventileinrichtung 23 angeordnet. Die erste Ventileinrichtung 23 ist vorgesehen derart angesteuert und/oder geschaltet zu werden, dass ein Durchgang von Prozessfluid von der zweiten Filtereinrichtung 3 zur Aufnahmeeinrichtung 15 für gebrauchtes Prozessfluid über den dritten Leitungsabschnitt 19 des Prozessfluidkreislaufs 5 zugelassen oder unterbunden wird.

Im vierten Leitungsabschnitt 21 ist stromabwärts der erste Filtereinrichtung 1 eine zweite Ventileinrichtung 25 angeordnet. Die zweite Ventileinrichtung 25 ist vorgesehen, derart angesteuert und/oder geschaltet zu werden, dass ein Durchgang von Prozessfluid von der ersten Filtereinrichtung 1 zur Aufnahmeeinrichtung 15 für gebrauchtes Prozessfluid über den vierten Leitungsabschnitt 21 des Prozessfluidkreislaufs 5 zugelassen oder unterbunden wird.

Während des Einsatzes der erfindungsgemäßen medizinischen Funktionseinrichtung 100, zum Beispiel zum Spülen und/oder Primen des extrakorporalen Blutkreislaufs 200 oder während einer extrakorporalen Blutbehandlung, wird Prozessfluid (Qin) aus der Prozessfluidquelle 11 entnommen und dem ersten Abschnitt 1a der ersten Filtereinrichtung 1 zugeführt.

In der ersten Filtereinrichtung 1 wird das Prozessfluid in der Regel, vorzugsweise, vollständig über die Hohlfasermembran 1c in den zweiten Abschnitt 1b der ersten Filtereinrichtung 1 filtriert.

Mittels der zweiten Ventileinrichtung 25, welche in bestimmten erfindungsgemäßen Ausführungsformen nicht vorgesehen ist oder zum hier beschriebenen Vorgehen nicht verwendet wird, kann eine periodische Abreinigung der Hohlfasermembran 1c vorgenommen werden, um im Falle einer Deckschichtbildung an der Hohlfasermembran 1c diese freizuspülen. Diese kann zu diesem Zweck, zumindest vorübergehend, ganz oder teilweise, geöffnet werden.

Der vierte Leitungsabschnitt 21 und die zweite Ventileinrichtung 25 der in Fig. 1 gezeigten Ausführung sind jedoch nicht zwingend erforderlich. In anderen Ausführungsformen der erfindungsgemäßen medizinischen Funktionseinrichtung 100 können sie daher weggelassen sein.

Die zweite Ventileinrichtung 25 ist in bestimmten Ausführungsformen als Proportionalventil ausgeführt.

Alternativ oder ergänzend zur zweiten Ventileinrichtung 25 ist in manchen Ausführungsformen der vorliegenden Erfindung eine okkludierende Pumpe vorgesehen.

In Ausführungsformen, in denen die zweite Ventileinrichtung 25 als Proportionalventil ausgeführt ist, kann ein Teilstrom des durch die erste Filtereinrichtung 1 strömenden Prozessfluids als Querstrom abgezweigt werden und zur kontinuierlichen Verringerung einer Deckschichtbildung an der Membran eingesetzt werden.

Das in der ersten Filtereinrichtung 1 filtrierte Prozessfluid (Qout) wird anschließend durch den zweiten Leitungsabschnitt 17 des Prozessfluidkreislaufs 5 in den Prozessfluid führenden Abschnitt 31 der zweiten Filtereinrichtung 3 geleitet.

Im Falle einer Blutbehandlung, zum Beispiel einer Dialysebehandlung, nimmt das Prozessfluid in der zweiten Filtereinrichtung 3 Toxine aus dem Blut auf und wird anschließend über den dritten Leitungsabschnitt 19 des Prozessfluidkreislaufs 5 in die Aufnahmeeinrichtung 15 für gebrauchtes Prozessfluid der Prozessfluidversorgungseinheit 13 (zum Beispiel Hydraulik einer Behandlungsvorrichtung, Bilanziersystem, Batchsystem mit ein oder zwei getrennten Behältern für die Versorgung mit frischem Prozessfluid und für die Aufnahme gebrauchten Prozessfluids) geleitet.

Durch vollständigen oder teilweisen Verschluss des dritten Leitungsabschnitts 19 mittels der ersten Ventileinrichtung 23 - oder alternativ einer okkludierenden Pumpe - kann das Prozessfluid über die Hohlfasermembran 35 in den Blut führenden Abschnitt 33 der zweiten Filtereinrichtung 3 geleitet, d.h. filtriert, werden.

Auf diese Weise kann durch geeignetes Schalten der ersten Ventileinrichtung 23 eine Umschaltung zwischen Behandlung, Primen, Blutrückgabe, Bolusgabe usw. auf vorteilhaft einfache und technisch wenig aufwendige Weise erreicht werden.

**Fig. 2** zeigt einen Abschnitt einer erfindungsgemäßen medizinischen Funktionseinrichtung 100 gemäß einer alternativen zweiten Ausführungsform.

Die erste Filtereinrichtung 1 und/oder die zweite Filtereinrichtung 3 sind analog der in Fig. 1 beschriebenen ersten Ausführungsform der erfindungsgemäßen medizinischen Funktionseinrichtung 100 ausgestaltet.

In der in Fig. 2 gezeigten Ausführungsform ist neben der ersten Fördereinrichtung 7 eine zweite Fördereinrichtung 27 angeordnet. Sie befindet sich im zweiten Leitungsabschnitt 17 des Prozessfluidkreislaufs 5 zwischen der ersten Filtereinrichtung 1 und der zweiten Filtereinrichtung 3 - stromabwärts der ersten Filtereinrichtung 1.

Die zweite Fördereinrichtung 27 kann in anderen Ausführungsformen auch an einer anderen Stelle als zwischen der ersten Filtereinrichtung 1 und der zweiten Filtereinrichtung 3 angeordnet sein. Beispielsweise kann die zweite Fördereinrichtung 27 in Strömungsrichtung des Prozessfluids stromaufwärts von der ersten Filtereinrichtung 1 angeordnet sein.

Der Prozessfluidkreislauf 5 weist eine Verzweigung 29 auf, an welcher der dritte Leitungsabschnitt 19 mit dem ersten Leitungsabschnitt 9 in Fluidverbindung zusammengeführt wird.

An der Verzweigung 29 wird aus der zweiten Filtereinrichtung 3 durch den dritten Leitungsabschnitt 19 strömendes gebrauchtes Prozessfluid mit aus der Prozessfluidquelle 11 durch den ersten Leitungsabschnitt 9 strömendem frischem Prozessfluid (Qfeed) zusammengeführt.

Während eines extrakorporalen Blutbehandlungsverfahrens strömt Prozessfluid von der Prozessfluidquelle 11 als Strom Qfeed - angetrieben durch die erste Fördereinrichtung 7 - in die erste Filtereinrichtung 1. Ein Teil hiervon tritt als Strom Qout aus der ersten Fördereinrichtung 1 aus. Nach Passieren der zweiten Fördereinrichtung 27 wird das Prozessfluid als Strom QD über den zweiten Leitungsabschnitt 17 in die zweite Filtereinrichtung 3 eingebracht.

In der zweiten Filtereinrichtung 3 wird das Prozessfluid, z.B. Dialysierflüssigkeit, beispielsweise bei einer Hämodialyse im Gegenstromprinzip an ebenfalls strömendem Blut vorbeigeführt. Nach Verlassen der zweiten Filtereinrichtung 3 gelangt das Prozessfluid in den dritten Leitungsabschnitt 19, wo es nach der Verzweigung 29 als Strom Qin erneut zur ersten Filtereinrichtung 1 geführt wird. Aus der ersten Filtereinrichtung 1 kann das Prozessfluid über den vierten Leitungsabschnitt 21 zur Aufnahmeeinrichtung 15 für gebrauchtes Prozessfluid zurückgeleitet werden. Es kann alternativ verworfen werden.

In manchen Ausführungsformen ist vorgesehen, gebrauchtes Prozessfluid über eine entsprechende Entsorgungsleitung (in Fig. 2 nicht gezeigt) und/oder eine Bilanziereinrichtung (in Fig. 2 nicht gezeigt) aus dem Prozessfluidkreislauf 5 abzuziehen. Die Menge des dem Prozessfluidkreislauf 5 abgezogenen Prozessfluids kann durch Zugabe frischen Prozessfluids aus der Prozessfluidquelle 11 über den ersten Leitungsabschnitt 9 ausgeglichen werden.

Zum Durchführen eines extrakorporalen Blutbehandlungs-Prozesses stellt das Betreiben der ersten Fördereinrichtung 7 zum Fördern des Prozessfluids durch den Prozessfluidkreislauf 5 eine ausreichende Förderung sicher. Die zweite Fördereinrichtung 27 muss daher während des Blutbehandlungs-Prozesses nicht notwendigerweise betrieben werden.

Zum Primen oder Befüllen des extrakorporalen Blutkreislaufs 200 wird Prozessfluid, zum Beispiel Dialysierflüssigkeit, von der Prozessfluidquelle 11, z.B. einer online-Dialysierflüssigkeitsquelle zur ersten Filtereinrichtung 1 gefördert.

In diesem ersten Schritt einer Kaskadenfiltration wird im Beispiel der Fig. 2 das Prozessfluid mittels eines Sterilfiltermoduls der ersten Filtereinrichtung 1 gefiltert: das Prozessfluid strömt durch die Hohlfasermembran 1c der ersten Filtereinrichtung 1 hindurch und wird hierbei gereinigt, insbesondere sterilfiltriert.

Wenn die zweite Ventileinrichtung 25 geschlossen ist, so findet an der ersten Filtereinrichtung 1 eine so genannte "dead end"-Filtration statt. Da der Flussweg über den Ausgang des ersten Abschnitts 1a der ersten Filtereinrichtung in den vierten Leitungsabschnitt 21 blockiert ist, muss das Prozessfluid die Hohlfasermembran 1c passieren.

Nach Verlassen der ersten Filtereinrichtung 1 mit einem Strom Qout passiert das Prozessfluid die Fördereinrichtung 27 und gelangt als Strom QD zur zweiten Filtereinrichtung 3.

In der zweiten Filtereinrichtung 3 kann mittels einer Druckdifferenz zwischen einem Prozessfluid führenden Abschnitt 31 und dem Hohlfasermembranlumen das Prozessfluid über die Hohlfasermembran 35 in diesem zweiten Schritt der Kaskadenfiltration in das Lumen des Blut führenden Abschnitts 33 der zweiten Filtereinrichtung 3 eintreten.

Die Druckdifferenz kann ein Transmembrandruckgefälle sein.

Ein Sperren des Durchflusses des Prozessfluids durch den dritten Leitungsabschnitt 19 mit Hilfe der ersten Ventileinrichtung 23 kann den Übertritt des Prozessfluids in den extrakorporalen Blutkreislauf 200 begünstigen.

Im extrakorporalen Blutkreislauf 200 kann das Prozessfluid zum Spülen entsprechender Blut führender Leitungen und/oder Einrichtungen genutzt werden.

Ein Sperren der zweiten Ventileinrichtung 25 kann zudem ein Abfließen von Prozessfluid aus der ersten Filtereinrichtung 1 zur Prozessfluidquelle 11 verhindern.

Die erfindungsgemäße externe medizinische Funktionseinrichtung kann vorteilhaft im Wesentlichen oder ausschließlich nur durch entsprechendes Schalten der ersten und zweiten Ventileinrichtung 23, 25 und/oder Betätigen der ersten Fördereinrichtung 7 zwischen einem Spül- oder Priming- und/oder Befüll-Prozess und Blutbehandlungs-Prozess wechseln.

**Bezugezeichenliste**

| **Bezugszseichen** | **Beschreibung** |
|---|---|
| 100 | Medizinische Funktionseinrichtung |
| 200 | Extrakorporaler Blutkreislauf |
| 1 | Erste Filtereinrichtung |
| la | erster Abschnitt der ersten Filtereinrichtung |
| 1b | zweiter Abschnitt der ersten Filtereinrichtung |
| 1c | Hohlfasermembran |
| 3 | Zweite Filtereinrichtung |
| 31 | Prozessfluid führender Abschnitt |
| 33 | Blut führender Abschnitt |
| 35 | Hohlfasermembran |
| 5 | Prozessfluidkreislauf |
| 7 | erste Fördereinrichtung |
| 9 | erster Leitungsabschnitt |
| 11 | Prozessfluidquelle |
| 13 | Prozessfluidversorgungseinheit |
| 15 | Aufnahmeeinrichtung für gebrauchtes Prozessfluid |
| 17 | zweiter Leitungsabschnitt |
| 19 | dritter Leitungsabschnitt |
| 21 | vierter Leitungsabschnitt |
| 23 | erste Ventileinrichtung |
| 25 | zweite Ventileinrichtung |
| 27 | zweite Fördereinrichtung |
| 29 | Verzweigung |

## Patentansprüche

1. Medizinische Funktionseinrichtung (100), vorgesehen und ausgestaltet zur einmaligen Verwendung in einem Verfahren zum extrakorporalen Behandeln des Bluts eines Patienten, ausgestaltet als Einweg-Disposable, und aufweisend
- wenigstens einen Prozessfluidkreislauf (5) oder Abschnitte desselben, jeweils vorgesehen zum Aufnehmen eines Prozessfluids;
- wenigstens eine erste Filtereinrichtung (1), welche innerhalb des Prozessfluidkreislaufs (5) oder eines Abschnitts hiervon angeordnet ist,
- wenigstens eine zweite Filtereinrichtung (3), welche innerhalb des Prozessfluidkreislaufs (5) oder eines Abschnitts hiervon angeordnet ist,
- wenigstens die erste Filtereinrichtung (1) vollständig oder abschnittsweise in die medizinische Funktionseinrichtung (100) integriert ist,
**dadurch gekennzeichnet, dass**
- die erste Filtereinrichtung (1) ein Sterilfilter ist und
- die zweite Filtereinrichtung (3) eine Blutfiltereinrichtung ist.

2. Medizinische Funktionseinrichtung (100) nach Anspruch 1, welche wenigstens eine Fördereinrichtung (27) aufweist, welche im Prozessfluidkreislauf (5) angeordnet ist, um das Prozessfluid im Prozessfluidkreislauf (5) oder in einem Abschnitt hiervon zu fördern.

3. Medizinische Funktionseinrichtung (100) nach Anspruch 1 oder 2, ferner aufweisend wenigstens einen extrakorporalen Blutkreislauf (200) oder Abschnitte desselben.

4. Medizinische Funktionseinrichtung (100) nach einem der vorangegangenen Ansprüche, welche zu ihrem Einsatz mit wenigstens einer Prozessfluidquelle (11) verbindbar oder verbunden ist, wobei die Prozessfluidquelle (11) in Strömungsrichtung des Prozessfluids stromaufwärts der ersten Filtereinrichtung (1) angeordnet ist.

5. Medizinische Funktionseinrichtung (100) nach einem der vorangegangenen Ansprüche, wobei wenigstens eine Absperreinrichtung und/oder eine Ventileinrichtung (23) und/oder eine okkludierende Pumpe, vorzugsweise stromabwärts der zweiten Filtereinrichtung (3), im Prozessfluidkreislauf (5) angeordnet ist.

6. Medizinische Funktionseinrichtung (100) nach einem der Ansprüche 1 bis 5, wobei wenigstens eine Fördereinrichtung, insbesondere eine Pumpe zur Proportionalregelung, stromabwärts der zweiten Filtereinrichtung (3) im Prozessfluidkreislauf (5) angeordnet ist.

7. Medizinische Funktionseinrichtung (100) nach einem der vorangegangenen Ansprüche, vorgesehen und/oder konfiguriert, um bei ihrer Ankoppelung oder Verbindung mit einer medizinischen Blutbehandlungsvorrichtung derart angesteuert zu werden, dass durch entsprechendes Schalten wenigstens einer Ventileinrichtung (23) im Prozessfluidkreislauf (5) und/oder Betätigen wenigstens einer der Fördereinrichtungen (7, 27) im Prozessfluidkreislauf (5) das Einleiten von Prozessfluid in den Prozessfluidkreislauf (5) und/oder in den extrakorporalen Blutkreislauf (200) veranlassbar ist.

8. Medizinische Funktionseinrichtung (100) nach einem der vorangegangenen Ansprüche, vorgesehen und/oder konfiguriert, um derart angesteuert zu werden, dass sie zwischen einem Spül- und/oder Befüll-Prozess und einem extrakorporalen Blutbehandlungs-Prozess schaltbar ist.

9. Medizinische Funktionseinrichtung (100) nach einem der Ansprüche 2 bis 8, wobei die Fördereinrichtung (27) vorgesehen und konfiguriert ist, um mittels wenigstens eines Aktors der medizinischen Behandlungsvorrichtung betrieben zu werden.

10. Medizinische Funktionseinrichtung (100) nach einem der Ansprüche 2 bis 9, wobei die Fördereinrichtung (27) ausgewählt ist aus Membranpumpen, peristaltischen Pumpen und/oder Impellerpumpen.

11. Medizinische Funktionseinrichtung (100) nach einem der vorangegangenen Ansprüche, wobei die erste Filtereinrichtung (1) und/oder die zweite Filtereinrichtung (3) als Hohlfaserfiltermodule und/oder mit Hohlfaserkapillarmembranen ausgestaltet sind.

12. Medizinische Funktionseinrichtung (100) nach einem der vorangegangenen Ansprüche, wobei stromabwärts der ersten Filtereinrichtung (1) eine zweite Ventileinrichtung (25) und/oder eine okkludierende Pumpeinrichtung angeordnet ist.

13. Verfahren zum Betreiben einer medizinischen Funktionseinrichtung (100) gemäß Anspruch 12 mit den Schritten:
- Bereitstellen einer medizinischen Funktionseinrichtung (100) gemäß Anspruch 12; und
- vollständiges oder teilweises Öffnen der zweiten Ventileinrichtung (25) und/oder der okkludierenden Pumpeinrichtung zum Reinigen der ersten Filtereinrichtung (1).

14. Medizinische Behandlungsvorrichtung, vorgesehen und konfiguriert zum Ansteuern wenigstens einer medizinischen Funktionseinrichtung (100) gemäß einem der Ansprüche 1 bis 12 und damit verbunden.

## Claims

1. A medical functional device (100) provided and configured for single use in a method of extracorporeally treating the blood of a patient, configured as one-way disposable, and comprising:
- at least one process fluid circuit (5) or sections thereof, each provided for receiving a process fluid;
- at least one first filter device (1) arranged within the process fluid circuit (5) or a section thereof,
- at least one second filter device (3) arranged within the process fluid circuit (5) or a section thereof,
- at least the first filter device (1) completely or partially integrated in the medical functional device (100),
**characterized in that**:
- the first filter device (1) is a sterile filter
and
- the second filter device (3) is a blood filter device.

2. The medical functional device (100) according to claim 1, comprising at least one conveying means (27) arranged in the process fluid circuit (5) for conveying the process fluid in the process fluid circuit (5) or in a section thereof.

3. The medical functional device (100) according to claim 1 or 2, further comprising at least one extracorporeal blood circuit (200) or sections thereof.

4. The medical functional device (100) according to anyone of the preceding claims, which is connectable or connected for use with at least one process fluid source (11), wherein the process fluid source (11) is arranged in the flow direction of the process fluid upstream of the first filter device (1) .

5. The medical functional device (100) according to anyone of the preceding claims, wherein at least one shut-off device and/or a valve device (23) and/or an occluding pump, is arranged, preferably downstream of the second filter device (3), in the process fluid circuit (5).

6. The medical functional device (100) according to anyone of claims 1 to 5, wherein at least one conveying means, in particular a pump for proportional control, is arranged downstream of the second filter device (3) in the process fluid circuit (5).

7. The medical functional device (100) according to anyone of the preceding claims, provided and/or configured to be driven, when coupled or connected with a medical blood treatment apparatus, such that by corresponding switching of at least one valve device (23) in the process fluid circuit (5) and/or by actuating at least one of the conveying means (7, 27) in the process fluid circuit (5), the introduction of the process fluid into the process fluid circuit (5) and/or into the extracorporeal blood circuit (200) is caused.

8. The medical functional device (100) according to anyone of the preceding claims, provided and/or configured to be driven, such that it is switchable between a rinsing and/or a filling process and an extracorporeal blood treatment process.

9. The medical functional device (100) according to anyone of the claims 2 to 8, wherein the conveying means (27) is provided and configured to be driven by means of at least one actuator of the medical treatment apparatus.

10. The medical functional device (100) according to anyone of claims 2 to 9, wherein the conveying means (27) is selected from diaphragm pumps, peristaltic pumps and/or impeller pumps.

11. The medical functional device (100) according to anyone of the preceding claims, wherein the first filter device (1) and/or the second filter device (3) are designed as hollow fibers filters modules and/or with hollow fibers capillary membranes.

12. The medical functional device (100) according to anyone of the preceding claims, wherein a second valve device (25) and/or an occluding pump device is arranged downstream of the first filter device (1).

13. A method of operating a medical functional device (100) according to claim 12 comprising the steps of:
- providing a medical functional device (100) according to claim 12; and
- complete or partial opening of the second valve device (25) and/or of the occluding pump device for cleaning the first filter device (1).

14. A medical treatment apparatus provided and configured to drive at least one medical functional device (100) according to anyone of the claims 1 to 12 and connected thereto.

## Revendications

1. Un dispositif fonctionnel médical (100) prévu et configuré pour un usage unique dans un procédé de traitement extracorporel du sang d'un patient, configuré en tant que matériel à usage unique et comprenant:
- au moins un circuit de fluide de traitement (5) ou des parties de celui-ci, chacune prévue pour recevoir un fluide de traitement;
- au moins un premier dispositif de filtration (1), agencé dans le circuit de fluide de traitement (5) ou dans une partie de celui-ci,
- au moins un second dispositif de filtration (3), agencé dans le circuit de fluide de traitement (5) ou dans une partie de celui-ci,
- au moins le premier dispositif de filtration (1), complètement ou partiellement intégré au dispositif fonctionnel médical (100),
**caractérisé en ce que** :
- le premier dispositif de filtration (1) est un filtre stérile
et
- le second dispositif de filtration (3) est un dispositif de filtration du sang.

2. Le dispositif fonctionnel médical (100) selon la première revendication, comprenant au moins un moyen d'acheminement (27) agencé dans le circuit de fluide de traitement (5) pour acheminer le fluide de traitement dans le circuit de fluide de traitement (5) ou dans une partie de celui-ci.

3. Le dispositif fonctionnel médical (100) selon la revendication 1 ou 2, comprenant en outre au moins un circuit sanguin extracorporel (200) ou des parties de celui-ci.

4. Le dispositif fonctionnel médical (100) selon l'une quelconque des revendications précédentes, connectable ou connecté pour son utilisation avec au moins une source de fluide de traitement (11), où la source de fluide de traitement (11) est agencée en amont du premier dispositif de filtration (1) dans la direction d'écoulement du fluide de traitement.

5. Le dispositif fonctionnel médical (100) selon l'une quelconque des revendications précédentes, où au moins un dispositif de fermeture et/ou un dispositif de vanne (23) et/ou une pompe occlusive, est/sont agencé (s) dans le circuit de fluide de traitement (5), de préférence en aval du second dispositif de filtration (3).

6. Le dispositif fonctionnel médical (100) selon l'une quelconque des revendications 1 à 5, où au moins un moyen d'acheminement, en particulier une pompe pour régulation proportionnelle, est agencé en aval du second dispositif de filtration (3) dans le circuit de fluide de traitement (5) .

7. Le dispositif fonctionnel médical (100) selon l'une quelconque des revendications précédentes, prévu et/ou configuré pour être commandé lors de son couplage ou de sa connexion avec un appareil médical de traitement du sang de telle sorte que par commutation correspondante d'au moins un dispositif de vanne (23) dans le circuit de fluide de traitement (5) et/ou par actionnement d'au moins un des moyens d'acheminement (7, 27) dans le circuit de fluide de traitement (5), l'introduction de fluide de traitement dans le circuit de fluide de traitement (5) et/ou dans le circuit sanguin extracorporel (200) soit engendrée.

8. Le dispositif fonctionnel médical (100) selon l'une quelconque des revendications précédentes, prévu et/ou configuré pour être commandé de telle sorte qu'il soit commutable entre un processus de rinçage et/ou un processus de remplissage et un processus de traitement du sang extracorporel.

9. Le dispositif fonctionnel médical (100) selon l'une quelconque des revendications 2 à 8, où le moyen d'acheminement (27) est prévu et configuré pour être actionné à l'aide d'au moins un actionneur de l'appareil de traitement médical.

10. Le dispositif fonctionnel médical (100) selon l'une quelconque des revendications 2 à 9, où le moyen d'acheminement (27) est choisi parmi un groupe comprenant des pompes à diaphragme, des pompes péristaltiques et/ou des pompes à impulseur.

11. Le dispositif fonctionnel médical (100) selon l'une quelconque des revendications précédentes, où le premier dispositif de filtration (1) et/ou le second dispositif de filtration (3) sont conçus comme des modules de filtres à fibres creuses et/ou avec des membranes à fibres creuses capillaires.

12. Le dispositif fonctionnel médical (100) selon l'une quelconque des revendications précédentes, où un second dispositif de vanne (25) et/ou un dispositif de pompe occlusive est agencé en aval du premier dispositif de filtration (1).

13. Un procédé de fonctionnement d'un dispositif fonctionnel médical (100) selon la revendication 12, comprenant les étapes suivantes :
- fournir un dispositif fonctionnel médical (100) selon la revendication 12; et
- ouvrir complètement ou partiellement le second dispositif de vanne (25) et/ou le dispositif de pompe occlusive pour le nettoyage du premier dispositif de filtration (1).

14. Un appareil de traitement médical prévu et configuré pour contrôler au moins un dispositif fonctionnel médical (100) selon l'une des revendication 1 à 12 et connecté à celui-ci.
